(19) Europäisches Patentamt
European Patent Office
Office européen des brevets

(11) **EP 2 447 868 A2**

(12) **EUROPEAN PATENT APPLICATION**

(43) Date of publication:
**02.05.2012  Bulletin 2012/18**

(51) Int Cl.:
***G06F 19/20*** *(2011.01)*

(21) Application number: **11184276.1**

(22) Date of filing: **07.10.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.10.2010  US 391338 P**

(71) Applicants:
• **F.Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO RS SE SI SK SM TR**
• **Roche Diagnostics GmbH**
  **68305 Mannheim (DE)**
  Designated Contracting States:
  **DE**

(72) Inventors:
• **Richmond, Todd**
  **Madison, WI 53719 (US)**
• **Jiang, Nan**
  **Wauwatosa, WI 53222 (US)**

(74) Representative: **Burger, Alexander**
  **Roche Diagnostics GmbH**
  **Patent Department (LPP.....6164)**
  **P.O.Box 11 52**
  **82372 Penzberg (DE)**

(54) **Methods of array data wave correction**

(57)    The invention relates to methods for correcting wave artifacts of hybridization signal data and for identifying copy number variations in a test sample.

EP 2 447 868 A2

**Description**

**Background of the Invention**

The invention relates to methods for correcting wave artifacts of hybridization signal data and for identifying copy number variations in a test sample.

Array-based technologies have significantly advanced the understanding of genomic and epigenomic phenomena and their variations. For example, comparative genomic hybridization (CGH) is a useful tool for analyzing genomic imbalances relating to copy number changes. ChIP-chip, which combines chromatin immunoprecipitation with microarray technology, can be used to identify protein binding sites. Array-based technologies can also be used to gather nucleic acid information with regard to sequence, nucleotide polymorphisms, and epigenetic modifications, such as methylation.

Typically, these methods involve labeling a test nucleic acid sample, hybridizing the labeled sample to immobilized probes on an array, detecting hybridization signals emitted by labeled test nucleic acids hybridized to the array probes, and analyzing the hybridization signal data to further a particular experimental objective. For example, CGH includes mixing differentially labeled test and control genomic samples and hybridizing the mixture to an array of defined probes that can span genomic areas of interest or the entire genome. If the test sample does not carry any copy number variations (CNVs) with regard to the control sample, the array hybridization signals for both samples are equal, i.e., the ratio between test and control sample equals 1. However, if the test sample contains a CNV, such as a deletion or duplication, the hybridization signal correlating to the test sample will be lower or higher than the control sample, depending on the nature of the CNV, i.e., the ratio will be >1 or <1. CGH can, thus, be used to identify CNVs by identifying genomic areas that have a signal ratio greater or smaller than 1.

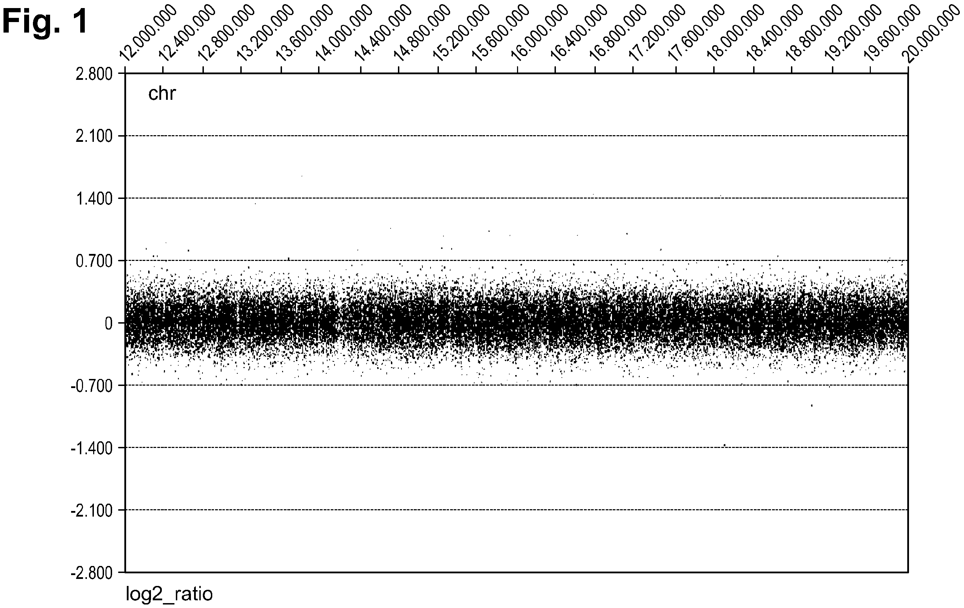

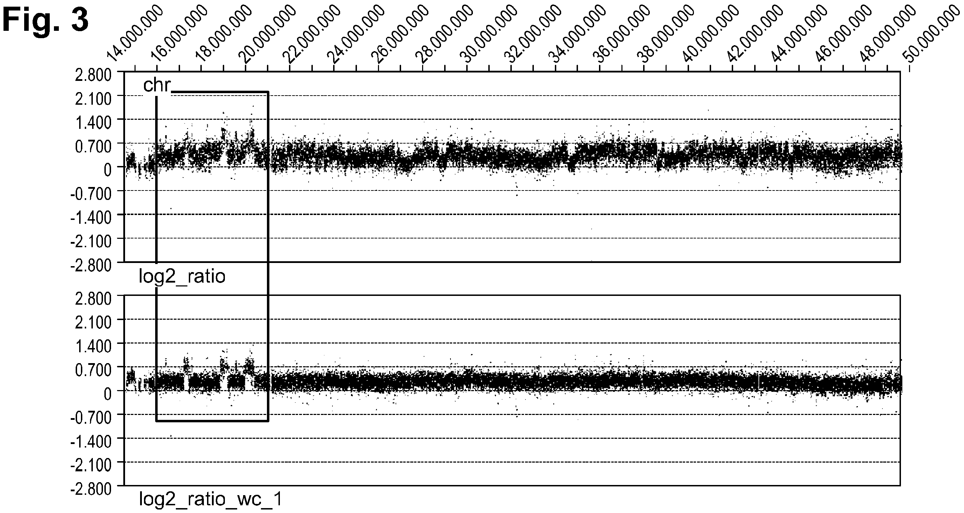

The results of array-based analysis can be graphically represented as scatter plots, each spot representing a signal relative to a genomic position that can aid analysis. In the case of CGH, results can be plotted as the log ratios of the test and control sample hybridization data or the individual signal data along the genomic coordinates (FIG. 1). In the absence of CNVs, ratio or signal values form a flat baseline (FIG. 1). Deviations from the flat baseline indicate CNVs, such as genomic insertions and deletions (FIG. 3, lower panel).

Recently, the inventors and others (e.g., Marioni et al., Genome Biol. 8 (2007) R228) identified technical artifacts impeding the analysis and interpretation of array-based data. Specifically, auto-correlated wave patterns can be observed in log2 ratio profiles along genomic DNA segments, i.e., oscillating increases and decreases of the hybridization signals giving rise to wave-like patterns. These wave-like patterns can impede analysis of hybridization data.

For example, for CGH, these wavelike patterns represent deviations from the expected flat baseline profile regardless of the presence or absence of CNVs in the analyzed genomic segment. These wave artifacts occur independently of the type of genomic samples or hybridization arrays used (Diskin et al., Nucleic Acids Res. 36 (2008) e126). Wave artifacts obstruct effective identification of CNVs during CGH analysis.

Marioni et al. reported that the wave artifacts of a bacterial artificial chromosome microarray were correlated with GC content of the targeted genome. However, Marioni et al. teaches removing the artifact by fitting a LOWESS curve on the log2 ratio-chromosomal position plot, rather than by correcting for GC content. van de Wiel et al. reported a method for wave artifact correction that employs log ratios from samples believed to have no CNVs using multi-variable regression (van de Wiel et al., Bioinformatics 25 (2009) 1099-1104). However, this method does not account for the confounding effects from large segmental shifts. Others considered GC content for correction of wave artifacts (Diskin et al., Nucleic Acids Research Vol. 36 (2008) e126; Lepretre et al., Nucleic Acids Research 38 (2010) e94). However, none of these methods accounts for the fact that large segmental shifts confound the correlation between GC content variations and the wave pattern, which can severely skew results. Thus, available methods for removing wave artifacts can introduce such an artifact due to skewed regression or remove signals created by true CNVs by "blind" LOWESS smoothing.

Accordingly, there remains a great need in the art for effective methods for identifying and removing wave artifacts without compromising hybridization data analysis.

**BRIEF SUMMARY**

The invention relates generally to methods for correcting auto-correlated wave artifacts of array signal data based on the guanine-cytosine (GC) content of the nucleic acids without compromising hybridization signal analysis.

In a first aspect, a method for correcting wave artifacts in array signal data is summarized as a method including the steps of (a) segmenting observed hybridization signal data of a test sample and a control sample that hybridize to a set of probes, each probe covering a probe-binding section of the control sample; (b) for each probe, determining at least one GC content value of at least one genomic region that comprises at least one probe-binding section; (c) performing multi-variable polynomial regression analysis using the at least one GC content value determined in step (b) as an explanatory variable and the observed hybridization signal data as a response variable, to produce predicted hybridization signal data for each probe; and (d) subtracting the predicted hybridization signal data from the observed hybridization signal data to obtain corrected hybridization signal data.

In some embodiments, steps (a) through (d) are performed several times using different sets of empirically determined log ratios, including log ratios from all probes in the chromosome, log ratios from probes in the longest segment in the chromosome, and log ratios from all probes of the chromosome, but with segmental shifts removed. Each analysis yields a candidate set of the corrected log ratios. The candidate set that is least noisy, i.e., shows the strongest evidence of correction, measured by mean square deviations from segment means, is selected for the final corrected log ratios.

In some embodiments, the genomic region spans the probe and additional regions flanking the probe.

The methods described herein are useful in a variety of applications, such as genetic research and clinical diagnostics, that use array-based hybridization methods, such as GCH, ChIP-chip, and methylation analysis.

These and other features, objects and advantages of the present invention will become better understood from the description that follows. In the description, reference is made to the accompanying drawings, which form a part hereof and in which there is shown by way of illustration, not limitation, embodiments of the invention. The description of preferred embodiments is not intended to limit the invention from covering all modifications, equivalents and alternatives. Reference should therefore be made to the claims recited herein for interpreting the scope of the invention.

## BRIEF DESCRIPTION OF THE DRAWINGS

The present invention will be better understood and features, aspects, and advantages other than those set forth above will become apparent when consideration is given to the following detailed description thereof. Such detailed description makes reference to the following drawings.

| | |
|---|---|
| **FIG.1** | illustrates array hybridization signal data along the genomic coordinates that is free of wave artifacts. |
| **FIG. 2** | illustrates array hybridization signal data with wave artifacts. |
| **FIG. 3** | illustrates hybridization signal data before and after wave artifact correction using equation (4) as the model. |
| **FIG. 4** | illustrates hybridization signal data before and after wave artifact correction using equation (4) as the model. The top two dot tracks represent log ratios on probes. The two line tracks represent segment means that are generated by segmentation algorithm and that are used to identify CNVs. The bottom dot track represents log GC content within 256 base pair flanking regions. The boxed area indicates shifts removed by the wave artifact correction. |
| **FIG. 5** | illustrates hybridization signal data before and after wave artifact correction using equation (4) as the model. The top two dot tracks represent log ratios on probes. The two line tracks represent segment means that are generated by segmentation algorithm and that are used to identify CNVs. The bottom dot track represents log GC content within 256 base pair flanking regions. The boxed area indicates shifts unaffected by the wave artifact correction. |
| **FIG. 6A-C** | illustrate the effect of wave correction on data waviness scores (FIG. 6A), signal noise (FIG. 6B), and segmentation (FIG. 6C). |
| **FIG. 7** | illustrates log hybridization signals from single channels data before and after wave artifact correction using equation (4) as the model. The first and third dot tracks represent log signals of the Cy3 and Cy5 channels before correction. The second and fourth dot tracks represent log signals of the Cy3 and Cy5 channels after correction. |
| **FIG. 8** | illustrates hybridization signal data before and after wave artifact correction using one GC content factor and using three GC content factors. From the top: first panel: uncorrected data; second panel: data corrected using only GC content within the region covered by the probe; third panel: data corrected using only GC content within the region of 256 base pairs upstream and downstream of the probe; fourth panel: data corrected using only GC content within the region of 256,000 base pairs upstream and downstream of the probe; fifth panel: data corrected using GC contents from within the region covered by the probe, within the region of 256 base pairs upstream and downstream of the probe, and within the region of 256,000 base pairs upstream and downstream of the probe in the regression model of equation (4). |
| **FIG.9** | illustrates using feature 2 of the algorithm in the wave artifact correction method. From the Top: first panel: uncorrected data; second panel: data corrected using equation (4) without applying feature 2. The correction is skewed due to the presence of large segmental shifts; third panel: data corrected using equation (4) together with feature 2. |
| **FIG. 10** | illustrates a graphic representation of one possible embodiment of the claimed methods. Rmsd = root mean square deviation. |
| **FIG. 11** | illustrates decreasing waviness scores after correction. Each point on the plot corresponds to an individual array. Waviness scores were normalized such that a score of 1 equals no waviness and values greater |

than 1 indicate waviness. Waviness scores were reduced for all arrays tested, with the largest reductions being observed for arrays with the highest initial waviness scores.

FIG. 12        illustrates decreasing noise scores after correction. Each point on the plot corresponds to an individual array.

While the present invention is susceptible to various modifications and alternative forms, exemplary embodiments thereof are shown by way of example in the drawings and are herein described in detail. It should be understood, however, that the description of exemplary embodiments is not intended to limit the invention to the particular forms disclosed, but on the contrary, the intention is to cover all modifications, equivalents, and alternatives falling within the scope of the invention as defined by the appended claims.

## DETAILED DESCRIPTION OF PREFERRED EMBODIMENTS

Unless defined otherwise, all technical and scientific terms used herein have the same meaning as commonly understood by one of ordinary skill in the art to which the invention pertains. Although any methods and materials similar or equivalent to those described herein can be used in the practice or testing of the present invention, the preferred methods and materials are now described.

The invention relates to methods for correcting wave artifacts in array signal data. As used herein, the term "wave" or "wave artifact" describes the undulating data profile of hybridization data along chromosome coordinates unrelated to phenomena under investigation. The present invention relates to the inventors' observation that wave pattern artifacts can be predicted using information from the genome being analyzed. The information includes three variables for each data point that vary in accordance with the waves. This information is used for advanced modeling to determine GC content influence. The model using this information includes segmentation of the data to overcome the problems caused by large segmental shifts.

Regardless of how the DNA was prepared or what array was used for hybridization analysis, the methods described herein begin with segmenting the hybridization data of a test and a control sample. One of skill in the art will recognize the advantageous efficiency of using an algorithm to perform segmentation. For example, any array CGH segmentation algorithm designed to find CNVs, can be used for segmenting CGH data. Let $i_1$, $i_2$, ... $i_{m-1}$ be the segmentation breakpoints, and further define $i_0$ =1 and $i_m$ = n. Under this notation, probes $i_0$ through $i_1$ make up the first segment. Probes $i_{s-1}$ + 1 through $i_s$ make up the s-th segment for s = 2,...m, where m is total number of segments made on the chromosome. Let $\overline{Y}_s$ be the segment mean for the s-th segment. According to the above notation,

$$\overline{Y}_s = \frac{1}{i_s - i_{s-1}} \sum_{i=i_{s-1}+1}^{i_s} Y_i \text{ for } s = 2,...m, \text{ and } \overline{Y}_s = \frac{1}{i_s - i_{s-1}+1} \sum_{i=i_{s-1}}^{i_s} Y_i \text{ for } s = 1. \qquad ...(1)$$

Because of the aforementioned relation between GC content and wave artifact occurrence, the GC content of defined genomic regions for each probe is determined. As used herein, "GC content" means the number of guanine-cytosine pairings within a genomic region. Each genomic region used to determine GC content comprises at least the probe-binding section of the particular probe, i.e., a section of the genome covered by the probe during hybridization. Thus, a genomic region can consist of only the probe-binding section or, alternatively, can include base pairs covered by the probe during hybridization and additional regions flanking each side of the probe-binding section. The size of the genomic region can be determined empirically to achieve optimal correction outcome. In a preferred embodiment, more than one genomic region for each probe is analyzed for GC content such that the probe-covered regions as well as near and distant neighboring regions are considered for assessing GC content influence. For example, the probe covered region with 0, 256, and 256000 base pairs on each side can be used for the purpose of this analysis. Let $X_{ij}$ be logarithm of GC content or logarithm of a linear transformation of GC content in flanking region j of size $l_j$ of probe i, j =1,2,...p. Corresponding to the above exemplary *specifications*, p = 3 and $l_1$ = 0, $l_2$=256, $l_3$=256000. In one particular embodiment,

$$X_{ij} = \log_2 (\text{proportion of GC bases in flanking region } j \text{ of probe } i + 2)$$

wherein $X_{ij}$ represents density of hydrogen bonds between complementary DNA strands in the genomic region around probe[i].

$$X_{ij} = \log_2(\text{proportion of GC bases in flanking region } j \text{ of probe } i + 2),$$

wherein $X_{ij}$ represents density of hydrogen bonds between complementary DNA strands in the genomic region around probe $i$.

The resulting GC content values of the various defined genomic regions are then used as explanatory variables in a multi-variable polynomial regression. The probe log ratios are used as the response variables. The regression analysis produces a model of predicted log ratios for each probe based on the GC content at and around the region covered by each probe. In a preferred embodiment the regression is performed on different sets of empirically determined hybridization signal data log ratios to ascertain parameters of the model. Such sets of empirically determined hybridization signal data log ratios include, for example, log ratios from all probes in the chromosome, log ratios from probes in the longest segment in the chromosome, and log ratios from all probes of the chromosome with prior removal of segmental shifts. In general, the model is described as:

$$Y_i = Y_i^{corr} + Y_i^{GC} \qquad \ldots(2)$$

where $Y_i^{corr}$ is the log ratio value when the waviness artifact does not exist, and the artifact part

$$Y_i^{GC} = f(X_{i1}, X_{i2}, \ldots, X_{ip}; \beta_0, \beta_1, \ldots, \beta_q) + \varepsilon_i \qquad \ldots(3)$$

is a polynomial of $X_{i1}, X_{i2}, \ldots, X_{ip}$ plus a random error term. Here $\beta_0, \beta_1, \ldots, \beta_q$ are the coefficients. A specific example of such polynomial is

$$Y_i^{GC} = \beta_0 + \beta_1 X_{i1} + \beta_2 X_{i2} + \beta_3 X_{i3} + \beta_4 X_{i1} X_{i2} + \beta_5 X_{i1} X_{i3} + \beta_6 X_{i2} X_{i3} + \beta_7 X_{i1} X_{i2} X_{i3} + \varepsilon_i$$
$$\ldots(4)$$

Coefficients $\beta_0, \beta_1, \ldots, \beta_q$ can be determined in several different methods: (1) by fitting the model to $\{Y_i, X_{ij}\}$, $i = 1, 2, \ldots n$; (2) by fitting the model to $\{Y_i, X_{ij}\}$, with i running through all probes in the longest segment in the chromosome; and (3) by fitting the model to $\{Y_i^0, X_{ij}\}$, $i = 1, 2, \ldots n$, where $Y_i^0$ is the difference between the probe's log ratio and its segment mean, $Y_i^0 = Y_i - \overline{Y}_s$, here $i$ runs through probes in segment $s$ for $s = 1, 2, \ldots m$.

From these data, a model is established with several candidate parameter sets of $\beta_0, \beta_1, \ldots, \beta_q$. Using each parameter set, a predicted waviness baseline $\{Y_i^{GC}\}$, $i = 1, 2, \ldots n$ is established. The empiric hybridization signal data is then corrected by subtracting the predicted waviness baseline from the empiric hybridization signal data, resulting in the corrected hybridization signal data, as is indicated by equation (2). Because each of the several candidate parameter sets are used for the correction, this step results in several candidate sets of the corrected log ratios. These candidate sets of the corrected log ratios are then compared. The set that is least noisy is selected as the final set of corrected log ratios. As used herein, "noise" means fluctuation of signals that are not attributable to segment shifts. The magnitude of noise can be measured, for example, by mean squared deviations from the segment means, defined as

$$R = \sqrt{\frac{1}{n-1} \sum_{s=1}^{m} \sum_{i \in \text{segment } s} (Y_i^{corr} - \overline{Y}_s^{corr})^2} \ .$$

In this equation, $\overline{Y}_s^{corr}$ is the segment mean of the corrected log ratios, which is calculated using equation (1), with $Y_i^{corr}$ in place of $Y_i$.

As described above, the novel methods include two stages. In the first stage, empiric hybridization data is fit in the model and the relevant parameters are determined. In the second stage, the model with the parameters determined in the first stage is applied to the hybridization data set to be corrected. The empiric hybridization data used in stage one need not include the entire data set to be corrected, i.e., portions of the hybridization data set to be corrected are sufficient. In fact, the empiric hybridization data used for the first stage need not be part of the hybridization data set to be corrected.

In one embodiment of the invention, three different sets of empiric hybridization data are used in stage one to determine the model parameters. For example, for a given data set to be corrected, the full segmented data set, the longest segment of the data set, and the full data set without segmental shifts can be used to determine parameters used for regression. All three models, each with its own parameter set, are then applied to the data set to be corrected. The model that produces the best, i.e. "least noisy," artifact correction is chosen for correction. Quality of correction can be assessed, for example, by calculating for each candidate of corrected data the standard deviation (SD) from segment means using segments from the uncorrected data. Optionally, the candidate of corrected data can be re-segmented prior to determining the SD. The smaller the SD, the better the correction.

In another embodiment, the waviness correction algorithm includes additional metrics to determine the model parameters. For example, a measure of waviness, referred to herein as "waviness score," can be used to select the best model between GC content and log ratios or log signals. Alternatively, or in addition, a measure of noise, referred to herein as "noise score," can be used during the selection step.

Thus, the described methods distinguish between segmental shifts and waviness by segmenting and correcting wave artifacts using inherent properties of the data, not by simply smoothing waves. The described methods can include multi-variable regression to incorporate GC content information from different flanking regions for wave pattern prediction. Further, in the described methods, the data set used to predict the wave pattern can be different from the data set to be corrected. The former can be a set that is derived from the latter with potentially confounding segmental shifts removed, or can be a subset of the latter that does not have such confounding shifts.

Further embodiments are included by the following items:

1. A method for correcting wave artifacts of hybridization signal data, the method comprising the steps of:

(a) segmenting observed hybridization signal data of a test and a control sample that hybridize to a set of probes, each probe covering a probe-binding section of the control sample;
(b) for each probe, determining at least one GC content value of at least one genomic region that comprises at least one probe-binding section;
(c) performing multi-variable polynomial regression analysis using the at least one GC content value determined in step (b) as an explanatory variable and the observed hybridization signal data as a response variable, to produce predicted hybridization signal data for each probe; and
(d) subtracting the predicted hybridization signal data from the observed hybridization signal data to obtain corrected hybridization signal data.

2. The method of item 1, wherein the genomic region is between about 50 and about 1,000,000 nucleotides in length.

3. The method of item 1, wherein the genomic region is about 572 nucleotides in length.

4. The method of item 1, wherein the genomic region is about 60 nucleotides in length.

5. The method of item 1, further comprising the step of empirically determining an optimal size of the genomic region.

6. The method of item 1, wherein the observed hybridization signal data is empirically determined.

7. The method of item 1, wherein the observed hybridization signal data of the test sample and the control sample comprises a log signal of the test sample and a log signal of the control sample.

8. The method of item 1, wherein the observed hybridization signal data of the test sample and the control sample comprises a log ratio of hybridization signal data of the test sample and the control sample.

9. The method of item 1, wherein the observed hybridization signal data is produced in a comparative genomic hybridization assay.

10. The method of item 9, wherein the data is segmented using a CGH segmentation algorithm.

11. The method of item 1, wherein the observed hybridization signal data is produced in a ChIP-chip assay.

12. The method of item 1, wherein the observed hybridization signal data is produced in a DNA methylation array assay.

13. A method for identifying copy number variations in a test sample, the method comprising the steps of:

(a) providing a set of probes that cover probe-binding segments of the test sample during hybridization;
(b) hybridizing to the probes a control sample labeled with a first label and a test sample labeled with a second label, such that observed hybridization signal data are produced;
(c) segmenting the observed hybridization signal data of the test and the control sample;
(d) for each probe, determining at least one GC content value of at least one genomic region that comprises at least one probe-binding section;
(e) performing multi-variable polynoimal regression analysis using the GC content values determined in step (d) as an explanatory variable and the observed hybridization signal data as a response variable, to produce predicted hybridization signal data for each probe; and
(f) subtracting the predicted hybridization signal data from the observed hybridization signal data to obtain corrected hybridization signal data.

14. The method of item 13, wherein the genomic region is between about 50 and about 1,000,000 nucleotides in length.

15. The method of item 13, wherein the genomic region is about 572 nucleotides in length.

16. The method of item 13, wherein the genomic region is about 60 nucleotides in length.

17. The method of item 13, wherein steps (c)-(f) are performed using a log signal of the test sample and a log signal of the control sample as the observed hybridization signal data.

18. The method of item 13, wherein steps (c)-(f) are performed using a log ratio of the hybridization data of the test and the control sample as the observed hybridization signal data.

19. The method of item 13, wherein the segmentation is performed using a CGH segmentation algorithm.

The invention will be more fully understood upon consideration of the following non-limiting Examples.

## EXAMPLES

### Example 1

### Correction of wave artifacts

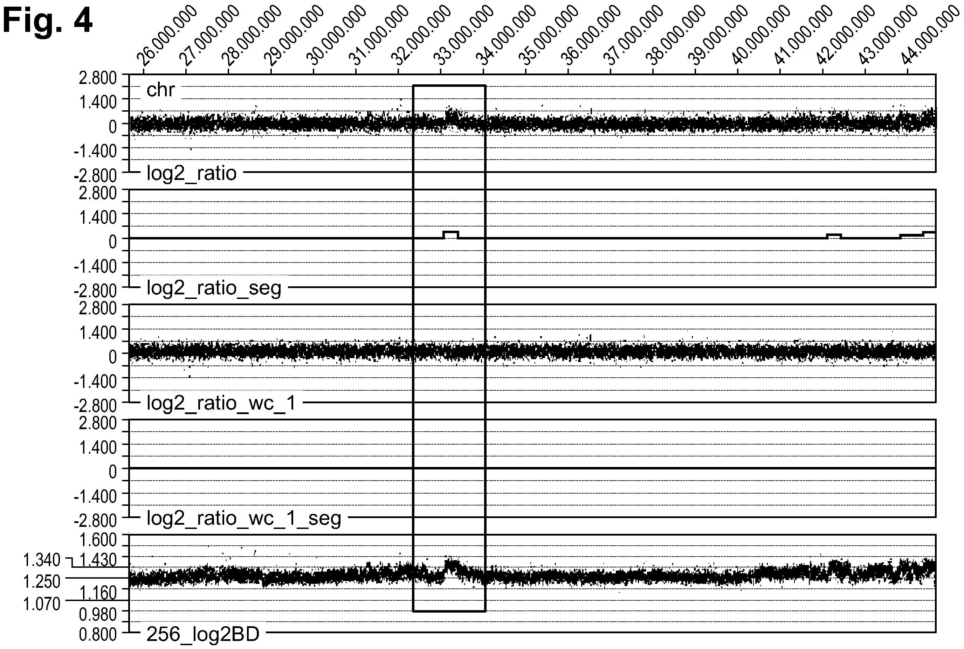

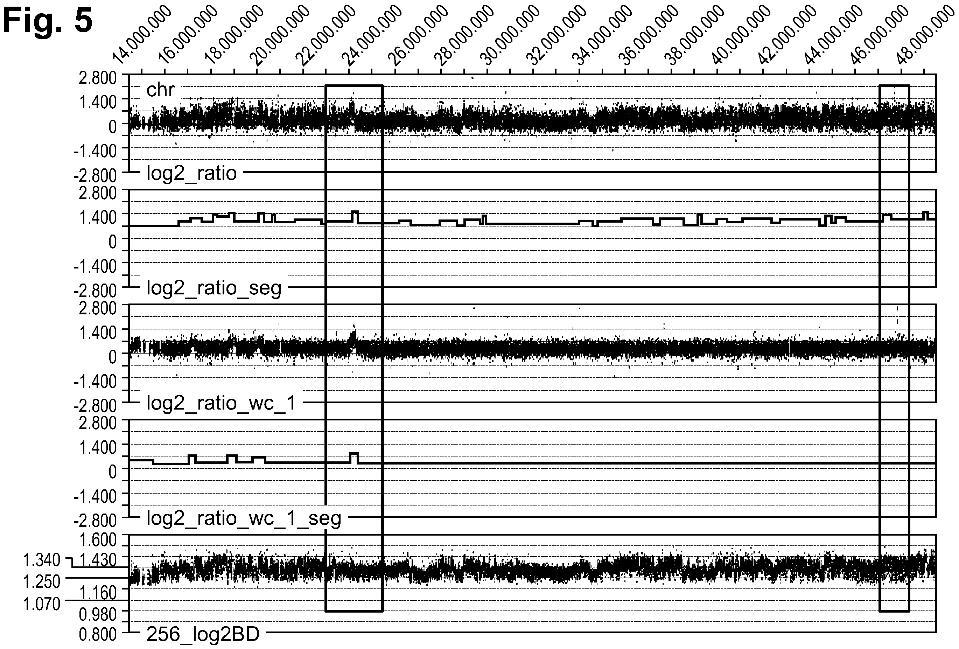

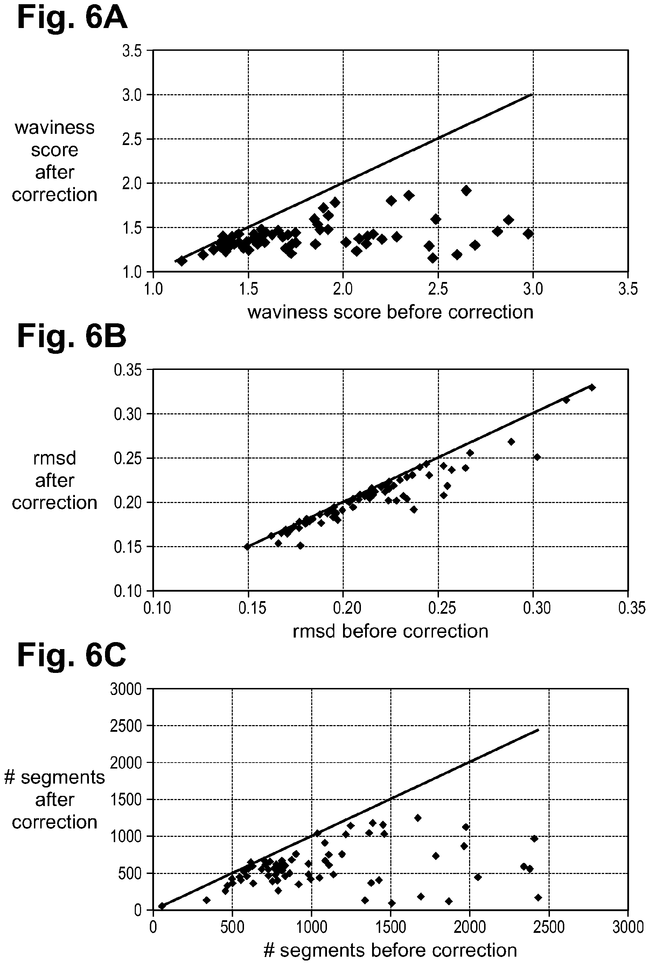

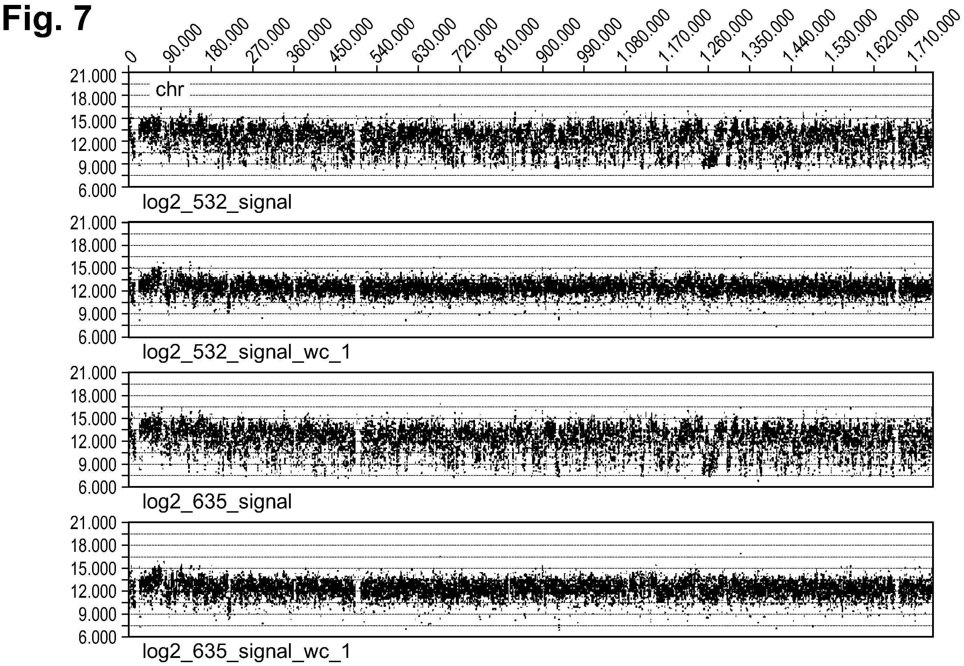

CGH was conducted under standard conditions and the hybridization signal data was expressed as log ratios of the Cy3 and Cy5 channels. The log ratios containing wave artifacts were corrected using equation (4) as the model using three GC content factors. Prior to correction, the hybridization signal data contained wave artifacts, obscuring shifts created by true CNVs (FIG. 3, top). Correction resulted in removal of wave artifacts, i.e., shifts in log ratio associated with GC content variations (FIG. 3, bottom, FIG. 4). The correction did not remove the shift in log ratio unrelated to GC content (FIG. 3, boxed area; FIG. 5, boxed area) suggesting that these shifts indicate true CNVs. The elevated data segments attributable to CNVs were more evident after correction.
In addition to improving identification of true CNVs, correction of wave artifacts also improved other parameters of hybridization data quality. Wave correction reduced overall waviness of the hybridization signal data, expressed as waviness score (FIG. 6A), reduced overall noise (FIG. 6B), and reduced the number of segments present in uncorrected data (FIG. 6C).
The correction method was also successfully applied to log signals of single channels, e.g., Cy3 and Cy5 channels (FIG. 7). Correction resulted in a marked reduction of the noise, in part, because part of the noise is attributable to GC-dependent wave artifacts that are removed during correction.

## Example 2

**Wave artifact correction using multiple GC content factors**

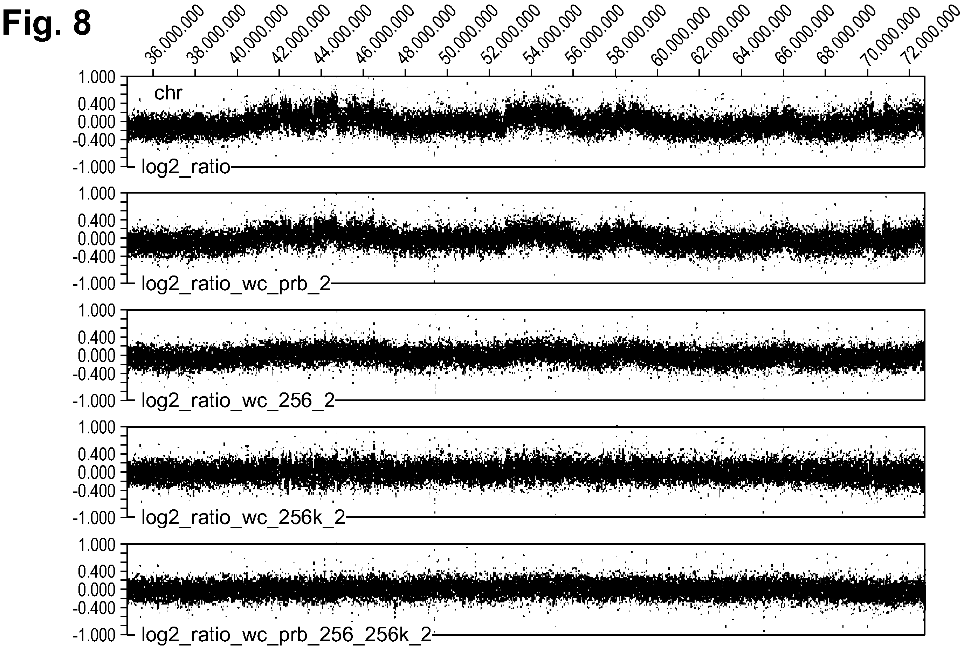

Using multiple GC content factors for correction results in superior wave artifact removal. Prior to correction, the hybridization signal data contained wave artifacts (FIG. 8, segMNT). When the data was corrected using the regression model of equation (4) with GC content data from either (1) within the region covered by the probe (FIG. 8, second dot track from top), (2) within the region of 256 base pairs upstream and downstream of the probe (FIG. 8, third dot track from top), or (3) within the region of 256000 base pairs upstream and downstream of the probe (FIG. 8, fourth dot track from top), some correction of wave artifacts occurred. Correction was superior when GC content information from all three regions was used in the regression model of equation (4) (FIG. 8, bottom dot track).

## Example 3

**Wave artifact correction using feature 2**

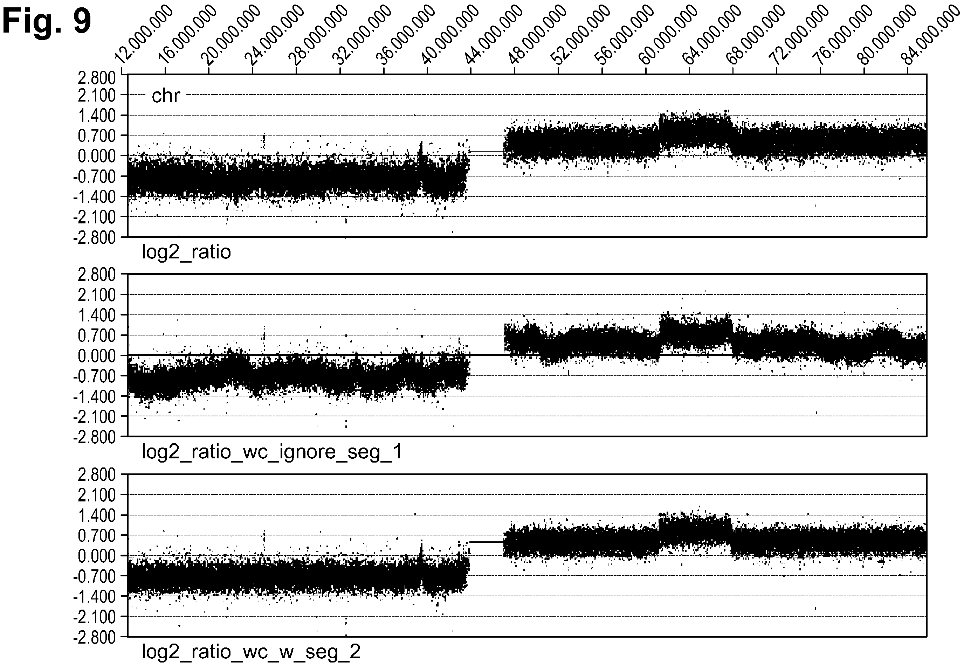

Figure 9 illustrates the advantage of using feature 2 of the algorithm in the wave artifact correction method. "Feature 2" refers to using several different sets of data, e.g., the full data set to be corrected, a subset of data consisting of data points that form the longest segment, and the full data set with segmental shifts removed, to determine several candidate parameter sets for the regression model. Each candidate parameter set is then applied to the model for correction and for picking the optimal result.

When the data was corrected using equation (4) without applying feature 2, the correction was skewed due to the presence of large segmental shifts (FIG. 9, second dot track). Superior correction was achieved when equation (4) was used together with feature 2 (FIG. 9, third dot track).

## Example 4

**Calculation of Waviness Scores**

In some embodiments of the claimed methods, a parameter of waviness ("waviness score") can be used to determine the best model for correction. A waviness score can be calculated using a variety of methods. In this example, the waviness score was derived by calculating the average absolute slope of the plot of log ratio or log signal versus genomic position. Because scatter plots often do not have a well defined slope, each plot was fit with a locally weighted scatter plot smoothing (LOWESS) curve and the slope of each curve was used as slope for the scattered plot. For CGH data, scales on x and y are several orders of magnitudes apart. Thus, to bring waviness scores to values of convenient range, slope values were multiplied by a factor of 10000000. The resulting slope has the unit of log2 ratio unit / 10000000 base pairs. If the slope is 1, the log 2 ratio drifts up or down by 1 unit in a genomic distance of 10000000 base pairs.

In this example, segment breakpoints were excluded from waviness score calculations to produce waviness scores that are scaled to mean absolute slopes of LOWESS curve fitting to the data. Consistent with standard LOWESS curve fitting methods (Cleveland, JASA 74 (1979) 829-836), all data points were considered for averaging except for potential breakpoints.

For LOWESS fitting, the fitting formula, the sliding window size, and the weight function were determined as follows:

Fitting formula:

$$y = f_i(x) = a_0 + a_1 x + a_2 x^2$$

Where x represents genomic position and y represents log ratio or log signal.

Sliding window size: 1000 - 2000 data points.

Weight function:

$$w(u) = (1 - u^3)^2 .$$

Where u is the scaled genomic distance to the center of the window.

Other fitting formula, sliding window sizes, and weight function can also be used.

### Example 5

**Wave artifact correction using waviness score**

Wave artifact correction was conducted essentially as illustrated by FIG. 10. CGH experiments were performed using 9 microarrays, each containing a plurality of probes that together cover the entire human genome. The waviness scores were calculated essentially as described in Example 4. Wave artifact correction resulted in a reduced waviness score (FIG. 11). Waviness scores were reduced for all arrays tested, with the greatest reduction in waviness being observed for high waviness scores of uncorrected data. Similarly, noise, measured by root mean square deviation (rmsd), was also decreased after wave artifact correction (FIG. 12). Rmsd is an indicator of random noise, high rmsd values indicating a high level of random noise in the data. As with waviness scores, greater reduction was observed on data with initially higher noise scores (FIG. 12).

### Claims

1. A method for correcting wave artifacts of hybridization signal data, the method comprising the steps of:

(a) segmenting observed hybridization signal data of a test and a control sample that hybridize to a set of probes, each probe covering a probe-binding section of the control sample;
(b) for each probe, determining at least one GC content value of at least one genomic region that comprises at least one probe-binding section;
(c) performing multi-variable polynomial regression analysis using the at least one GC content value determined in step (b) as an explanatory variable and the observed hybridization signal data as a response variable, to produce predicted hybridization signal data for each probe; and
(d) subtracting the predicted hybridization signal data from the observed hybridization signal data to obtain corrected hybridization signal data.

2. The method of Claim 1, wherein the genomic region is between about 50 and about 1,000,000 nucleotides in length.

3. The method of Claim 1, wherein the genomic region is about 572 nucleotides in length.

4. The method of Claim 1, wherein the genomic region is about 60 nucleotides in length.

5. The method of Claim 1, further comprising the step of empirically determining an optimal size of the genomic region.

6. The method of Claim 1, wherein the observed hybridization signal data is empirically determined.

7. The method of Claim 1, wherein the observed hybridization signal data of the test sample and the control sample comprises a log signal of the test sample and a log signal of the control sample.

8. The method of Claim 1, wherein the observed hybridization signal data of the test sample and the control sample comprises a log ratio of hybridization signal data of the test sample and the control sample.

9. The method of Claim 1, wherein the observed hybridization signal data is produced in a comparative genomic hybridization assay.

10. The method of Claim 9, wherein the data is segmented using a CGH segmentation algorithm.

11. A method for identifying copy number variations in a test sample, the method comprising the steps of:

(a) providing a set of probes that cover probe-binding segments of the test sample during hybridization;

(b) hybridizing to the probes a control sample labeled with a first label and a test sample labeled with a second label, such that observed hybridization signal data are produced;

(c) segmenting the observed hybridization signal data of the test and the control sample;

(d) for each probe, determining at least one GC content value of at least one genomic region that comprises at least one probe-binding section;

(e) performing multi-variable polynomial regression analysis using the GC content values determined in step (d) as an explanatory variable and the observed hybridization signal data as a response variable, to produce predicted hybridization signal data for each probe; and

(f) subtracting the predicted hybridization signal data from the observed hybridization signal data to obtain corrected hybridization signal data.

12. The method of Claim 11, wherein the genomic region is between about 50 and about 1,000,000 nucleotides in length.

13. The method of Claim 11, wherein the genomic region is about 572 nucleotides in length.

14. The method of Claim 11, wherein the genomic region is about 60 nucleotides in length.

15. The method of Claim 11, wherein steps (c)-(f) are performed using a log signal of the test sample and a log signal of the control sample as the observed hybridization signal data.

Fig. 1

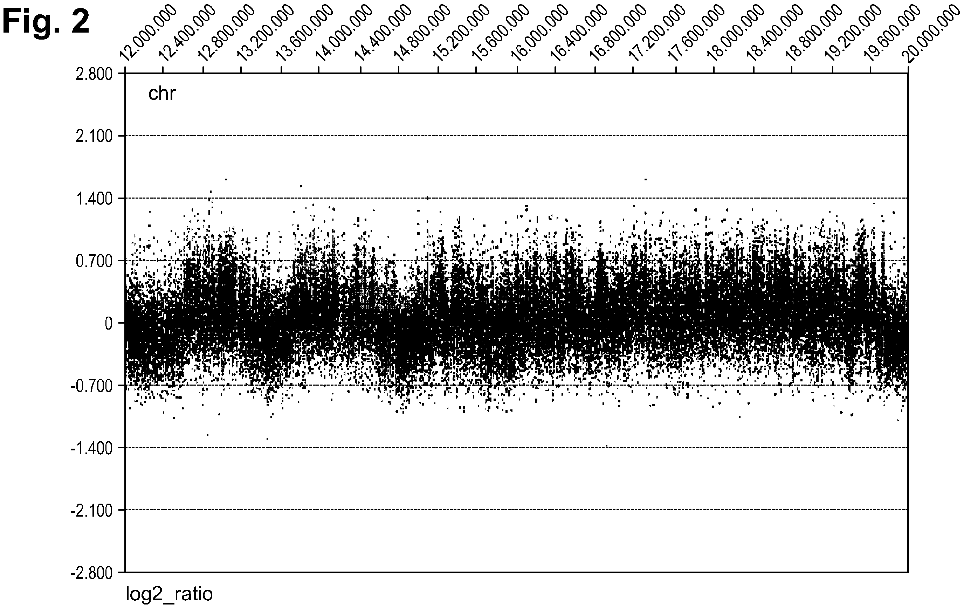

# Fig. 2

Fig. 3

# Fig. 4

EP 2 447 868 A2

Fig. 5

EP 2 447 868 A2

# Fig. 6A

# Fig. 6B

# Fig. 6C

# Fig. 7

log2_532_signal

log2_532_signal_wc_1

log2_635_signal

log2_635_signal_wc_1

# Fig. 8

Fig. 9

chr

log2_ratio

log2_ratio_wc_ignore_seg_1

log2_ratio_wc_w_seg_2

EP 2 447 868 A2

EP 2 447 868 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MARIONI et al.** *Genome Biol.,* 2007, vol. 8, R228
- **DISKIN et al.** *Nucleic Acids Res.,* 2008, vol. 36, 126
- **VAN DE WIEL et al.** *Bioinformatics,* 2009, vol. 25, 1099-1104
- **DISKIN et al.** *Nucleic Acids Research,* 2008, vol. 36, 126
- **LEPRETRE et al.** *Nucleic Acids Research,* 2010, vol. 38, 94
- **CLEVELAND.** *JASA,* 1979, vol. 74, 829-836